# FASCICULE DE BREVET EUROPEEN

(11) **EP 1 471 881 B1**
(45) Date de publication et mention de la délivrance du brevet: **15.10.2008**
(21) Numéro de dépôt: 03715039.8
(22) Date de dépôt: 22.01.2003
(51) Int. Cl.: A61K 8/44, A61K 31/41, A61K 31/55, A61Q 17/04, A61Q 19/02, C07C 233/49, C07C 233/51

(54) **UTILISATION D'UN COMPOSE INACTIVANT LA PROTEINE KINASE A DANS UNE COMPOSITION CONTENANT UN MILIEU COSMETIQUEMENT ACCEPTABLE, POUR ECLAIRCIR LA PEAU**
VERWENDUNG EINER PROTEINKINASE A-INAKTIVIERENDEN VERBINDUNG IN EINER KOSMETISCH VERTRÄGLICHEN ZUSAMMENSETZUNG ZUR AUFHELLUNG DER HAUT
USE OF A COMPOUND INACTIVATING THE KINASE A PROTEIN IN A COMPOSITION CONTAINING A COSMETICALLY ACCEPTABLE MEDIUM IN ORDER TO LIGHTEN THE SKIN

(30) Priorité: 25.01.2002 FR 0200925
(43) Date de publication de la demande: 03.11.2004
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: STOLTZ, Corinne, F-94320 Thiais (FR); GARCIA, Christine, F-81100 Castres (FR)
(74) Mandataire: Conan, Philippe Claude
(86) Numéro de dépôt international: PCT/FR2003/000210
(87) Numéro de publication internationale: WO 2003/061768

(56) Documents cités:
- WO-A-99/04757
- DATABASE CAPLUS [en ligne] CHEMICAL ABSTRACTS SERVICE, COLUMBUS, OHIO, US; retrieved from STN Database accession no. 1994:563706 XP002221803 & JP 06 157284 A (ADOBANSUTO SUKIN RISAACHI KENK) 3 juin 1994 (1994-06-03)
- DATABASE CAPLUS [en ligne] CHEMICAL ABSTRACTS SERVICE, COLUMBUS, OHIO, US; retrieved from STN Database accession no. 1996:271458 XP002221804 -& CHEMICAL ABSTRACTS, vol. 124, no. 24, 10 juin 1996 (1996-06-10) Columbus, Ohio, US; abstract no. 325007, XP002221802 & JP 08 053332 A (KAO CORP)
- DATABASE CAPLUS [en ligne] CHEMICAL ABSTRACTS SERVICE, COLUMBUS, OHIO, US; retrieved from STN Database accession no. 2002:441174 XP002221805 & JP 2002 167319 A (ASAHI KASEI CORP) 11 juin 2002 (2002-06-11)
- DATABASE CAPLUS [en ligne] CHEMICAL ABSTRACTS SERVICE, COLUMBUS, OHIO, US; retrieved from STN Database accession no. 1975:103149 XP002221806 & JP 49 093521 A (MORINAGA MILK INDUSTRY CO LTD) 5 septembre 1974 (1974-09-05)
- R. BUSCÀ ET AL: PIGMENT CELL RES, vol. 13, no. 2, 2000, pages 60-69, XP002221800
- M.E. HADLEY ET AL: ANNALS OF THE NEW YORK ACADEMY OF SCIENCES, vol. 680, 1993, pages 424-439, XP001040669
- A. LEYDET ET AL: J. MED. CHEM., vol. 39, no. 8, 1996, pages 1626-1634, XP002221801

## Description

La présente invention a pour objet une nouvelle utilisation d'actifs cosmétiques pour éclaircir la peau.

La plupart des formulations cosmétiques dépigmentantes commercialisées, sont à base d'acide kojique, d'arbutine ou de magnésium ascorbyl phosphate.

Le document JP06157284A décrit une composition cosmétique pour éclaircir la peau contenant par exemple le composé (Z, Z, Z)-N-(1-oxo-9, 12, 15-octadecatrienyl)-L—phenylalanine et le composé (Z, Z, Z)-N-(1-oxo-9, 12, 15-octadecatrienyl)-L—valine.

Le document JP08053332A décrit une composition cosmétique pour éclaircir la peau, contenant par exemple le sel de monosodique du N-(2-ethyl-1-oxohexyl)-L-tryptophane.

Le document JP49093521 décrit une composition destinée à un usage cosmétique, contenant par exemple comme agent actif le composé 1-(1-oxo-10-undecenyl)-L-proline.

L'article de J. Leydet and al. (J. Med. Chem., vol. 39, n°8, 1996, pages 1626-1634) décrit le N-( ω-undecylenoyl) phenylalanine ainsi que son activité thérapeutique et des compositions à usage pharmaceutique le contenant.

Les inventeurs se sont intéressés au développement de nouveaux actifs dépigmentants qui aient une meilleure compatibilité avec la peau que ceux de l'état de la technique. Ils ont mis en évidence que les molécules inactivant la protéine Kinase A, engendrait une dépigmentation de la peau que l'on attribuait jusqu'à aujourd'hui, à la seule inhibition de l'enzyme Tyrosinase phosphorylée.

C'est pourquoi, selon un premier aspect, l'invention a pour objet, l'utilisation de N-(ω-undécylènoyl) phénylalanine inactivant la protéine Kinase A dans une composition contenant un milieu cosmétiquement acceptable, pour éclaircir la peau.

La relation entre l'activité éclaircissante de la peau et l'inactivation de la protéine Kinase A (PKA), peut s'expliquer par le mécanisme biochimique suivant :

L'inhibition de la protéines Kinase A induit une moindre activation de la tyrosinase, du fait de la moindre transformation de cette dernière en tyrosinase phosphorylée ; cette moindre activation de la tyrosinase entraîne la diminution de la synthèse de mélanine d'où découle la dépigmentation de la peau.

Par composé inactivant la protéine Kinase A, on désigne notamment, tout composé qui, par incubation de la protéine Kinase A en présence d'adénosine triphosphate et d'une protéine pouvant être phosphorylée, telle que par exemple l'Histone H1, inhibe sa phosphorylation, avec un pourcentage d'inhibition supérieur ou égal à 10 %, plus particulièrement avec un pourcentage d'inhibition supérieur ou égal à 25 % et de préférence supérieur ou égal à 50 %.

Le composé (Ia) fait partie de la classe des composés de formule (I) : 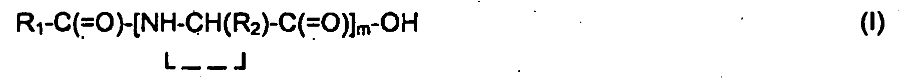 ou ses sels, dans laquelle R₁ représente la chaîne caractérisante d'un acide gras, saturé ou insaturé, linéaire ou ramifié, comportant de 3 à 30 atomes de carbone, R₂ représente la chaîne caractérisante d'un acide aminé et m est compris entre 1 et 50, ou d'un mélange desdits composés de formule (I) ou de leurs sels, dans une composition contenant un milieu cosmétiquement acceptable, pour éclaircir la peau.

Le composé de formule (I) telle que définie ci-dessus, peut être sous forme d'acide libre ou sous forme partiellement ou totalement salifiée. Lorsque le composé de formule (I) est sous forme salifiée, Il s'agit notamment de sels alcalins tels que les sels de sodium, de potassium ou de lithium, de sels alcalino-terreux tels que les sels de calcium, de magnésium ou de strontium ; de sel d'ammonium ou de sel d'un aminoalcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium.

Dans l'exposé suivant, par composé de formule (I), on entend composé de formule (I) sous forme libre ou sous forme partiellement ou totalement salifiée.

L'expression "chaîne caractérisante" utilisée pour définir les radicaux R₁ et R₂, désigne la chaîne principale non fonctionnelle de l'acide gras ou de l'acide aminé considéré.

Ainsi, pour un acide gras répondant à la formule générale R₁-C(=O)-OH, la chaîne caractérisante sera la chaîne représentée par R₁.

R₁-C(=O)- représente le radical, undécylènoyle.

Pour un acide aminé représenté par la formule générale (IIIa) :

H₂N-CH(R₂)-C(=O)-OH (IIIa),

comme pour un acide aminé cyclique représenté par la formule (IIIb) : 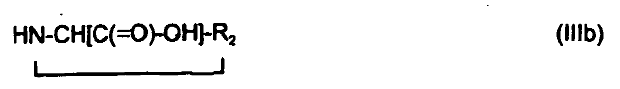 la chaîne caractérisant sera la chaîne représentée par R₂.

R₂ représente notamment la chaîne caractérisante de l'acide aminé .

Selon une autre variante particulière de la présente invention, on utilise un seul composé de formule (I), telle que définie précédemment, dans la composition contenant le milieu cosmétiquement acceptable..

Selon une autre variante particulière de la présente invention, on utilise un mélange de composés de formule (I) telle que définie précédemment et, plus particulièrement,
ou bien un mélange de composés de formules (I) comportant toutes le même fragment R₁-C(=O),
ou bien un mélange de composés de formules (I) dans lesquelles m est égal à 1 et comportant toutes le même fragment

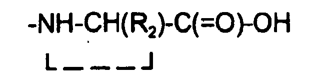

Les composés de formules (I) sont généralement obtenus par N-acylation de composés de formules (IIIa) ou (IIIb), telles que définies précédemment ou de leurs sels.

Lorsqu'il s'agit d'un mélange de composés de formules (1), il est par exemple obtenu par N-acylation du mélange d'acides aminés résultant de l'hydrolyse totale ou partielle de protéines de toutes origines.

Ces protéines peuvent être d'origine animale, telles que, par exemple, le collagène, l'élastine, la protéine de chair de poisson, la gélatine de poissons, la kératine ou la caséine, d'origine végétale, comme les protéines de céréales, de fleurs ou de fruits, telles que par exemple, les protéines issues du soja, du tournesol, de l'avoine, du blé, du maïs, de l'orge, de la pomme de terre, du lupin, de la féverole, de l'amande douce, du kiwi, de la mangue ou de la pomme ; il peut s'agir aussi de protéines obtenues à partir de chorelles (algues unicellulaires), d'algues roses, de levures ou de la soie.

Cette hydrolyse est réalisée par exemple, par chauffage à des températures comprises entre 60 et 130°C d'une protéine placée dans un milieu acide ou alcalin.

Cette hydrolyse peut également être réalisée par voie enzymatique avec une protéase, couplée éventuellement à une post-hydrolyse alcaline ou acide. Quand m est supérieur à 1, R₂ représente une seule même chaîne ou bien plusieurs chaînes caractérisant différents acides aminés, selon la protéine hydrolysée et le degré d'hydrolyse.

Les aminogrammes de quelques protéines d'origine végétales sont consignés dans le tableau suivant :

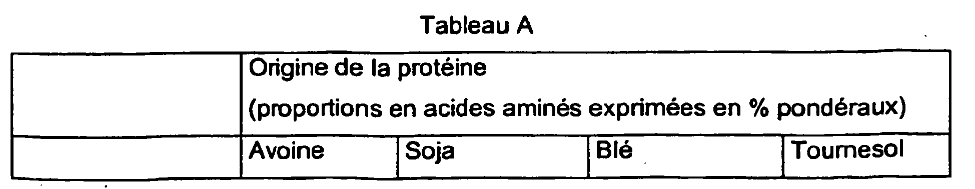

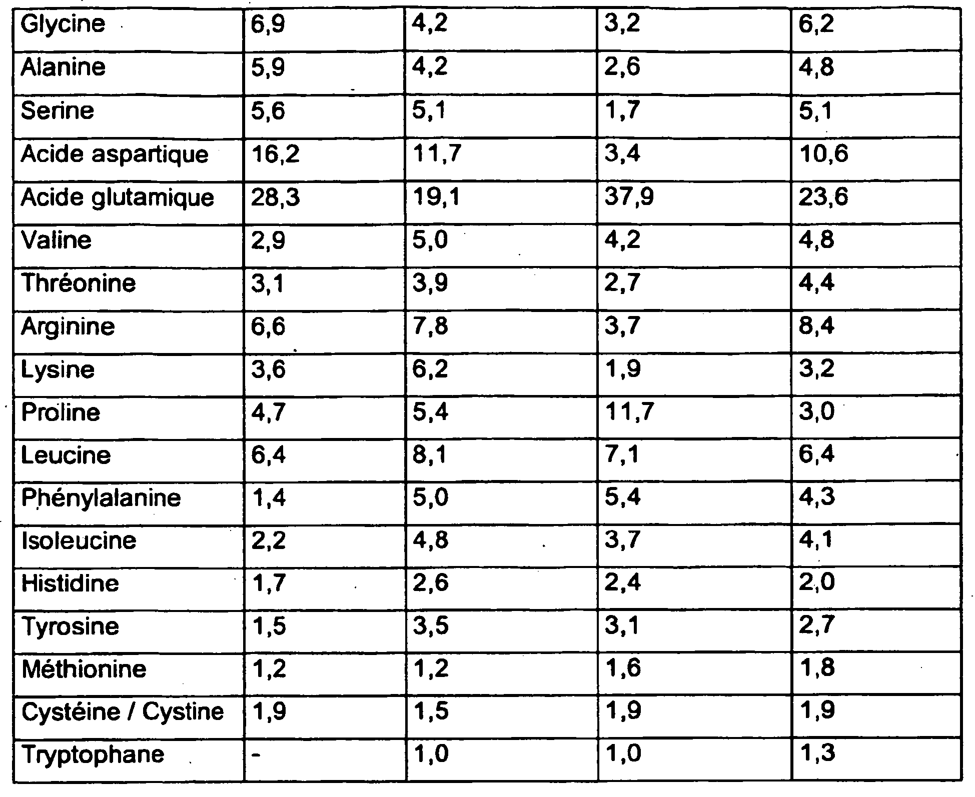

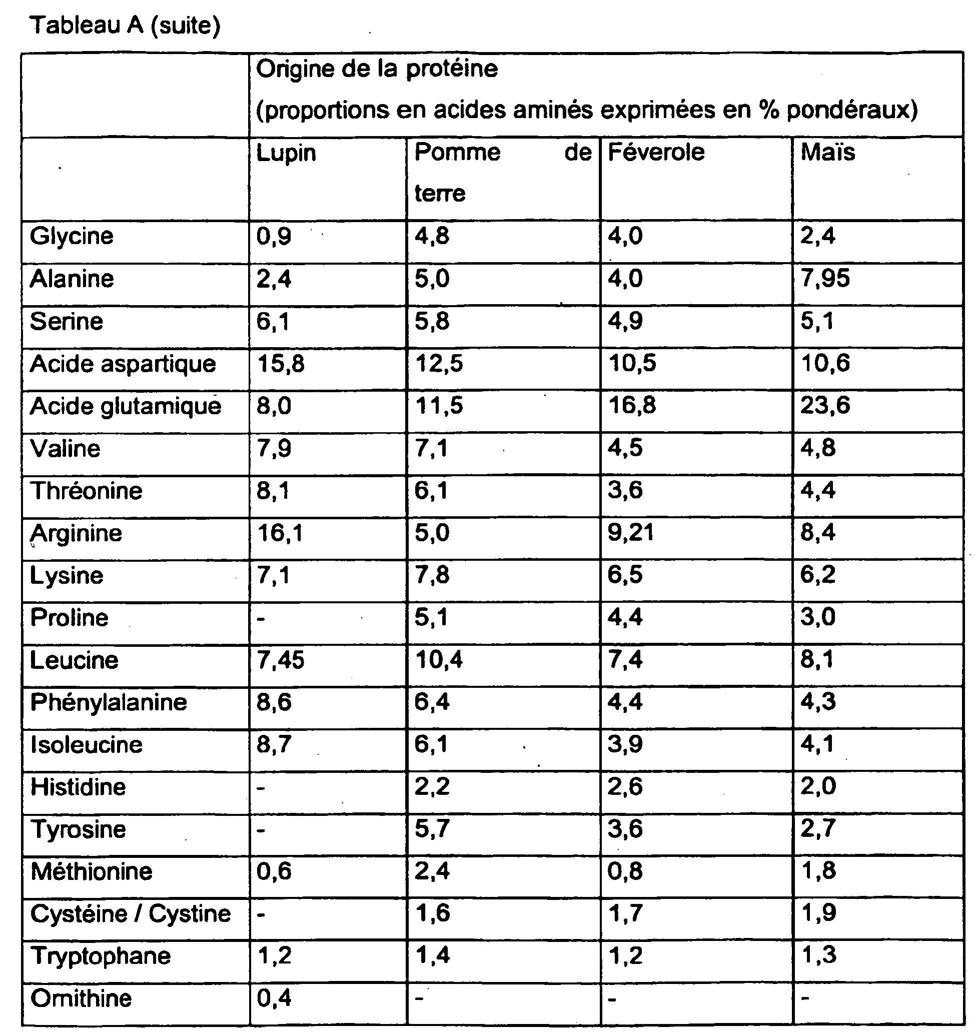

La réaction d'acylation est connue de l'homme du métier. Elle est décrite par exemple dans la demande internationale publiée sous le numéro WO 98/09611. Elle est mise en oeuvre indifféremment sur un acide aminé ou sur un mélange d'acides aminés. L'agent d'acylation consiste généralement en un dérivé activé d'un acide carboxylique de formule R₁C(=O)-OH, tel qu'un un anhydride symétrique de cet acide ou un halogénure d'acide comme le chlorure d'acide ou le bromure d'acide. Il peut aussi consister en un mélange de dérivés activés d'acides carboxyliques issus d'huiles ou graisses naturelles d'origine animales ou végétales telles que les huiles de coprah, de palmiste, de palme, de soja, de colza, de maïs, le suif de boeuf, l'huile spermaceti ou l'huile de hareng.

L'invention a aussi pour objet, un procédé de traitement non thérapeutique de la peau destiné à l'éclaircir, caractérisé en ce que l'on y applique une composition contenant un milieu cosmétiquement acceptable et une quantité efficace d'au moins le composé N-(ω-undécylénoyl) phénylalanine inactivant la protéine Kinase A.

Dans les compositions définies ci-dessus, le composé inactivant la protéine Kinase A est généralement mis en oeuvre en une quantité comprise entre 0,01 % et 10 % de leur poids, plus particulièrement entre 0,1 % à 5 % de leurs poids, et tout particulièrement entre 1 % et 5 % de leurs poids.

Selon un autre aspect particulier, l'invention a pour objet, l'utilisation telle que définie précédemment, caractérisé en ce que le composé inactivant la protéine Kinase A, inactive aussi l'adénylate cyclase.

La relation entre l'activité éclaircissante de la peau et l'inactivation l'adénylate cyclase, peut s'expliquer par le mécanisme biochimique suivant :

L'inactivation de l'adénylate cyclase entraîne une moindre transformation de l'ATP en AMP cyclique intracellulaire ; la diminution du taux d'AMP cyclique entraîne une inhibition de la protéine Kinase A (PKA) ; l'inhibition de la protéine Kinase A induit une moindre activation de la tyrosinase du fait de la moindre transformation de cette dernière en tyrosinase phosphorylée ; cette moindre activation de la tyrosinase entraîne la diminution de la synthèse de mélanine d'où découle la dépigmentation de la peau.

Par composé inactivant l'adénylate cyclase, on désigne notamment, dans le cadre de la présente invention, tout composé qui, par incubation de cet enzyme en présence d'adénosine triphosphate, inhibe sa transformation en adénosine monophosphate cyclique, avec un pourcentage d'inhibition supérieur ou égal à 10 %, plus particulièrement avec un pourcentage d'inhibition supérieur ou égal à 25 % et de préférence supérieur ou égal à 50 %.

Les composé inactivant l'adénylate cyclase contenus dans ladite composition, sont plus particulièrement choisis parmi les composés de formule (I) telle que définie précédemment ou leurs sels. et tout particulièrement parmi les composés de formule (I) telle que définie précédemment, dans laquelle R,-C(=O) est choisi parmi les radicaux octanoyle ou ω-undécylènoyle et dans laquelle dans au moins un des restes

-HN-CH(R₂)-C(=O)- (IIIa),

ou 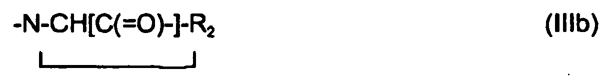 R₂ représente la chaîne caractérisante de la phénylalanine.

L'invention a aussi pour objet un procédé tel que défini précédemment, caractérisé en ce que l'on applique sur la peau, une composition contenant un milieu cosmétiquement acceptable et une quantité efficace d'au moins le composé N-(ω-undécylènoyl) phénylalanine inactivant la protéine Kinase A et l'adénylate cyclase. ainsi qu'une composition pharmaceutique telle que définie précédemment, caractérisée en ce qu'elle contient une quantité efficace d'au moins le composé N-(ω-undécylènoyl) phénylalanine inactivant la protéine Kinase A et l'adénylate cyclase.

Selon un autre aspect particulier, l'invention a pour objet, l'utilisation telle que définie précédemment, caractérisée en ce que le composé inactivant la protéine Kinase A et l'adénylate cyclase, est un composé présentant une affinité vis à vis du récepteur de la Mélanocyte Spécifique Hormone (α-MSH).

La relation entre l'activité éclaircissante de la peau et l'affinité vis à vis du récepteur de l'α-MSH, peut s'expliquer par le mécanisme biochimique suivant :

La compétition entre l'hormone α-MSH et la molécule présentant une affinité vis à vis du récepteur α-MSH, entraîne un moindre taux de fixation de ladite hormone sur les récepteurs cellulaires ; cette compétition a pour conséquence une inhibition de l'activité de l'adénylate cyclase qui entraîne une moindre transformation de l'ATP en AMP cyclique intracellulaire ; la diminution du taux d'AMP cyclique entraîne une inhibition de l'enzyme Protéine Kinase A (PKA) ; l'inhibition de la Protéine Kinase A induit une moindre activation de la tyrosinase du fait de la moindre transformation de cette dernière en tyrosinase phosphorylée ; cette moindre activation de la tyrosinase entraîne la diminution de la synthèse de mélanine d'où découle la dépigmentation de la peau. C'est l'ensemble de ces inhibitions successives qui témoignent du caractère antagoniste de l'α-MSH. des composés de l'invention.

Par composé présentant une affinité vis à vis du récepteur de la Mélanocyte Spécifique Hormone, l'α-MSH, on désigne dans le cadre de la présente invention, tout composé qui déplace la liaison spécifique d'un ligand radioactif comme le Nucléoside diphosphate-α-Mélanocyte Spécifique Hormone ([¹²⁵I]NDP-α-MSH) sur le récepteur de type 1 de l'α-Mélanocyte Spécifique Hormone (α-MSH), dénommé récepteur MC1R, avec un pourcentage d'inhibition supérieur ou égal à 10 %, plus particulièrement avec un pourcentage d'inhibition supérieur ou égal à 25 % et de préférence supérieur ou égal à 50 %.

Les antagonistes de la Mélanocyte Spécifique Hormone, contenus dans ladite composition, sont plus particulièrement choisis parmi les composés de formule (I) tel que définie précédemment ou leurs sels.

L'invention a aussi pour objet un procédé tel que défini précédemment, caractérisé en ce que l'on applique sur la peau, une composition contenant un milieu cosmétiquement acceptable et une quantité efficace d'au moins le composé N-(ω-undécylènoyl) phénylalanine inactivant la protéine Kinase A et l'adénylate cyclase, qui est antagoniste de la Mélanocyte Spécifique Hormone, ainsi qu'une composition pharmaceutique telle que définie précédemment, caractérisée en ce qu'elle contient une quantité efficace d'au moins le composé N-(ω-undécylènoyl) phénylalanine inactivant la protéine Kinase A et l'adénylate cyclase, qui est antagoniste de la Mélanocyte Spécifique Hormone.

Comme le montrent les exemples suivants, les composés mis en oeuvre dans les traitements cosmétiques ou thérapeutiques définis précédemment, se caractérisent de façon inattendue, par une activité éclaircissante de la peau supérieure aux compositions de l'état de la technique. Ils sont donc de façon générale appropriés aux traitements destinés à éclaircir la peau notamment par dépigmentation et plus particulièrement pour faire disparaître ou pour atténuer les taches colorées apparaissant sur les peaux âgées.

Les compositions mises en oeuvre dans lesdits traitements, se présentent généralement sous forme de solutions aqueuses ou hydroalcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements, sont administrées au sujet sous les formes classiques utilisées en cosmétique et en pharmacie ; il s'agit plus particulièrement des administrations topique, orale ou parentérale.

De façon générale, les composés de formule (I) inactivant la protéine Kinase A, éventuellement, l'adénylate cyclase et éventuellement antagonistes de la Mélanocyte Spécifique Hormone, qui sont mis oeuvre dans l'invention objet de la présente demande de brevet, telle que définie précédemment, sont associés à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, comme par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer au composé de formule (I), on peut citer, les paraffines, les isoparaffines, les huiles blanches minérales, les huiles végétales, les huiles animales, les huiles de synthèse, les huiles siliconées et les huiles fluorées ; et plus particulièrement :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales éthoxylées ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Comme autre matière grasse que l'on peut associer à cet actif, on peut citer les alcools gras ou les acides gras.

Parmi les polymères épaississants et/ou émulsionnant utilisés dans la présente invention, il y a par exemple, les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate commercialisés sous les dénominations CARBOPOL^{™} Ultrez^{™} 10, PEMULEN^{™} TR1, PEMULEN^{™} TR2, SIMULGEL^{™} A, SIMULGEL^{™} NS, SIMULGEL^{™} EPG, SIMULGEL^{™} EG, LUVIGEL^{™} EM, SALCARE^{™} SC91, SALCARE^{™} SC92, SALCARE^{™} SC95, SALCARE^{™} SC96, FLOCARE^{™} ET100, HISPAGEL^{™}, SEPIGEL^{™} 305, SEPIGEL^{™} 501, SEPIGEL^{™} 502, FLOCARE^{™} ET58, STABILEZE^{™} 06 ; les hydrocolloïdes d'origine végétale ou biosynthétique comme par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les cires utilisables dans le cadre de la présente invention, on peut citer par exemple la cire d'abeille ; la cire de carnauba ; la cire de candelilla ; la cire d'ouricoury ; la cire du Japon ; la cire de fibre de liège ou de carine à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Parmi les émulsionnants utilisables dans le cadre de la présente invention, on peut citer par exemple les acides gras ; les acides gras éthoxylés ; les esters d'acide gras et de sorbitol ; les esters d'acides gras éthoxylés ; les polysorbates ; les esters de polyglycérol ; les alcools gras éthoxylés ; les esters de sucrose ; les alkylpolyglycosides ; les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de principe actif que l'on peut associer au composé de formule (I), afin d'en potentialiser par synergie ses propriétés, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate les extraits de polyphénols, les extraits de raisin, les extraits de pin, les extraits de vin, les extraits d'olives, les extraits de mare, les protéines N-acylées, les peptide N-acylés, les acides aminés N-acylés, les hydrolysâts partiels de protéines N-acylés, les acides aminés, les peptides, les hydrolysâts totaux de protéines, les hydrolysâts partiels de protéines, les polyols (comme par exemple, la glycérine, le butylène glycol ...), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides comme par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja comme par exemple, la Raffermine^{™}, les extraits de blé comme par exemple la Tensine^{™} ou la Gliadine^{™}, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général , les lipides tels que les céramides ou les phospholipides, les actifs ayant une action amincissante comme par exemple, la caféine ou ses dérivés, les actifs ayant une activité anti-microbienne ou une action purifiante vis à vis des peaux grasses tels que le Lipacide^{™} PVB, les actifs ayant une propriété énergisante ou stimulante comme le Sepitonic^{™} M3 ou le Physiogényl^{™} le panthénol et ses dérivés comme le Sepicap^{™} MP, les actifs anti-age comme que Sepilift^{™} DPHP, le Lipacide^{™} PVB, le Sepivinol^{™}, le Sepivital^{™}, les actifs hydratants comme le SEPICALM^{™} S, le SEPICALM^{™} VG et le lipacide^{™} DPHP, les actifs anti-age " anti-photo vieillissement ", les actifs protecteurs de l'intégrité de la jonction dermo-épidermique, les actifs augmentant la synthèse des composants de la matrice extracellulaire.

Comme filtre solaire que l'on peut incorporer dans la composition selon l'invention, on peut citer tous ceux figurant dans la directive cosmétique 76/768/CEE modifiée annexe VII.

Selon un dernier aspect de la présente invention, celle-ci a pour objet une émulsion cosmétique contenant le N-(ω-undécylènoyl) phénylalanine de formule : 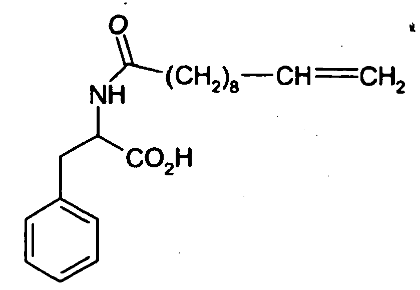 caractérisée en ce qu'elle en contient entre 0,01 % et 10 % de leurs poids, plus particulièrement entre 0,1 % à 5 % de leurs poids, et tout particulièrement entre 1 % et 5 % de leurs poids.

L'étude expérimentale suivante illustre l'invention sans toutefois la limiter.

### Evaluation in vitro de l'activité dépigmentante de l'undécylènoyl phénylalanine

L'objectif de cette étude a été de mettre en évidence l'activité dépigmentante du N-undécylènoyl phénylalanine, selon un mécanisme impliquant l'effet antagoniste de la molécule sur le récepteur de type 1 de l'α-Mélanocyte Spécifique Hormone (α-MSH), dénommé récepteur MC1 R. Ce type de récepteur pharmacologique est principalement rencontré dans les mélanocytes.

Le contrôle de la mélanogénèse à partir de ce récepteur, est représenté sur la figure 1. Il implique notamment l'adénylate cyclase, l'AMPc, la protéine kinase A et la tyrosinase. En se fixant sur le récepteur MC1R, l'α-MSH stimule la sous-unité α de la protéine G Stimulatrice (Prot GαS). Cette dernière active l'enzyme adénylate cyclase qui convertit l'adénosine triphosphate (ATP) en adénosine monophosphate cyclique (AMPc). L'AMPc active les protéines kinase A (PK A), qui transforment la tyrosinase en tyrosinase phosphorylée qui stimule la mélanogénèse.

Dans une première étape, l'étude à donc consisté à évaluer les capacités de fixation du N-undécylènoyl phénylalanine sur les récepteurs MC1R, que l'on rencontre dans les mélanocytes.

Dans une seconde étape, l'effet du N-undécylènoyl phénylalanine a été évalué sur les activités de l'adénylate cyclase, de la protéine kinase A et de la tyrosinase.

Dans une troisième étape, l'activité dépigmentante du N-undécylènoyl phénylalanine a été déterminée in vitro sur des cultures de mélanocytes de la lignée B16/F1 par mesure des contenus intra- et extracellulaires en mélanine et par mesure de l'activité tyrosinase.

Dans une quatrième étape, l'activité dépigmentante du N-undécylènoyl phénylalanine a été évaluée dans un modèle d'épidermes humains reconstruits pigmentés (phototype IV) afin de tester l'efficacité du produit en conditions réelles d'application (application topique du produit formulé).

Les effets du produit ont été comparés à ceux observés à ceux de différents produits dépigmentants de référence, l'hydroquinone, l'acide kojique et l'arbutine.

### 1 - Etude d'affinité sur les récepteurs MC1R

On a comparé l'affinité du N-undécylènoyl phénylalanine, de l'acide kojique et de l'arbutine.

Les récepteurs MC1R sont isolés à partir des membranes cellulaires de mélanocytes murins de la lignée B16/F1 par la méthode décrite dans : Siegrist W., Oestreicher M., Stutz M., Girard J. and Eberle A.E. ; J. Recep. Res., 8, 1988, 323-343".

Le N-undécylènoyl phénylalanine, l'arbutine et l'acide kojique sont dilués à une concentration de 10 mg / ml dans une solution aqueuse de soude décinormale. Ils sont testés chacun séparément aux concentrations de 0,1 mg / ml et 1 mg / ml. La soude n'a pas d'effet sur le paramètre étudié.

Les récepteurs MC1 R sont incubés, en présence ou en absence de ces produits, avec un ligand radioactif marqué à l'iode 125, le Nucléoside diphosphate-α-Mélanocyte Spécifique Hormone [¹²⁵I]NDP-α-MSH à la concentration de 0,05 nM, pendant 90 minutes à 22°C.

Des cultures témoins sont incubées, en absence de produit, et en présence du ligand radioactif. Chaque essai est réalisé trois fois.

Après 90 minutes d'incubation, les membranes cellulaires sont rapidement filtrées et les filtres sont lavés plusieurs fois par du tampon froid. La quantité de ligand radioactif fixé aux récepteurs MC1 R est mesurée avec un compteur à scintillation (Topcount, PACKARD).

Les résultats consignés dans le tableau suivant, sont les moyennes des trois essais réalisées pour chacun des produits; Ils sont exprimés en pourcentage de liaison spécifique par rapport au groupe témoin et en pourcentage d'inhibition de cette liaison.

| Produits Testés | Activité par rapport au témoin . | | Inhibition de la liaison spécifique par les produits testés | |
|---|---|---|---|---|
| | à 0,1 mg/ml | à 1 mg/ml | à 0,1 mg/ml | à 1 mg/ml |
| Arbutine | 100,80 ± 0,55 | 96,30 ± 4,16 | 0 % | 3,7% |
| Acide kojique | 104,50 ± 1,38 | 124,00 ± 1,87 | 0 % | 0 % |
| N-undécylènoyl phénylalanine | 57,70 ± 2,38 | 4,20 ± 0,86 | 42.3% | 95.8% |

Les résultats mettent en évidence qu'aux concentrations testées, ni l'arbutine, ni l'acide kojique, qui sont les composés dépigmentants de référence, ne déplace la liaison spécifique du ligand, le [¹²⁵I]NDP-α-MSH ; au contraire le N-undécylènoyl phénylalanine déplace respectivement de 42 % et 96 % la liaison du [¹²⁵I]NDP-α-MSH au récepteurs MC1R.

### 2 - Etude de l'activation de l'adénylate cyclase

On a comparé l'influence du N-undécylènoyl phénylalanine, de l'acide kojique et de l'arbutine sur la conversion de l'ATP en AMPc par un dosage radioimmunologique

L'adénylate cyclase qui convertit l'ATP en AMPc, est extraite de cerveaux de rats, par la méthode décrite dans "Salamon Y., Londos C. and Rodbell M. ; Anal. Biochem., 58, 1974, 541-548" ; elle est ensuite activée par 10 µM de forskoline.

Le N-undécylènoyl phénylalanine, l'arbutine et l'acide kojique sont dilués à une concentration de 10 mg / ml dans une solution aqueuse de soude décinormale. Ils sont testés chacun séparément à la concentration de 1 mg / ml. La soude n'a pas d'effet sur le paramètre étudié.

L'enzyme activée est incubée, en présence ou en absence de ces produits, et en présence d'ATP à 0,5 mM, pendant 30 minutes à 30°C.

Des cultures témoins sont incubées, en absence de produit, et en présence d'ATP. Chaque essai est réalisé trois fois.

Après 30 minutes d'incubation, la quantité d'AMPc produit est évaluée par un dosage radioimmunologique réalisé à l'aide d'un kit commercial ; la radioactivité est mesurée avec un compteur à scintillation (Topcount, PACKARD), une moindre radioactivité traduisant une moindre activation de l'adénylate cyclase.

Les résultats consignés dans le tableau suivant, sont les moyennes des trois essais réalisées pour chacun des produits; Ils sont exprimés en pourcentage d'activité enzymatique par rapport au groupe témoin et en pourcentage d'inhibition.

| Produits testés | Activité enzymatique par rapport au témoin | Inhibition de l'activité de l'adénylate cyclase. |
|---|---|---|
| Arbutine | 109,7 ± 4,6 % | 0 % |
| acide kojique | 45,0 ± 6,5 % | 55 % |
| N-undécylènoyl phénylalanine | -16,0 ± 0,5 % | 100 % |

Les résultats mettent en évidence qu'à 1 mg / ml, alors que l'arbutine n'induit aucun effet sur cet enzyme, l'acide kojique induit un effet moyen et le N-undécylènoyl phénylalanine une inactivation complète.

### 3 - Etude de l'activité de la protéine kinase A

On a comparé l'influence du N-undécylènoyl phénylalanine, de l'acide kojique et de l'arbutine sur la phosphorylation de la tyrosinase par la protéine kinase A (PK A).

La protéine kinase A est extraite de cerveaux de boeuf par la méthode décrite dans : "Chijiwa T., Mishima A., Hagiwara M., Sano M., Hayashi K., Inoue T., Naito K., Shioka T., Hidaka H.; J.Biol.Chem., 265, 1990,5267-5272" . Puis elle est activée par 3 µM de d'AMPc.

Le N-undécylènoyl phénylalanine, l'arbutine et l'acide kojique sont dilués à une concentration de 10 mg / ml dans une solution aqueuse de soude décinormale. Il sont testés chacun séparément à la concentration de 1 mg / ml. La soude n'a pas d'effet sur le paramètre étudié.

L'enzyme activée est incubée, en présence ou en absence de ces produits, et en présence d'ATP radioactif marqué au ³³P ([γ-³³P]ATP) et de 200 µg / ml d'histone H₁, pendant 20 minutes à 30°C.

Des cultures témoins sont incubées, en absence de produit, et en présence d'ATP radioactif et d'histone H₁. Chaque essai est réalisé trois fois.

Après 20 minutes d'incubation, la quantité d'histone H, phosphorylée marquée au ³³P est mesurée avec un compteur à scintillation (Topcount, PACKARD), une moindre radioactivité traduisant une moindre activation de la protéine kinase A.

Les résultats consignés dans le tableau suivant, sont les moyennes des trois essais réalisées pour chacun des produits; Ils sont exprimés en pourcentage d'activité enzymatique par rapport au groupe témoin et en pourcentage d'inhibition.

| Produits testés | Activité enzymatique par rapport au témoin. | Inhibition de l'activité de la protéine kinase A. |
|---|---|---|
| Arbutine | 90,9 ± 8,4 % | 9,1 % |
| acide kojique | 113,3 ± 5,0 % | 0 % |
| N-undécylènoyl phénylalanine | -0,4 ± 0,3 % | 100 % |

Les résultats mettent en évidence qu'à 1 mg / ml le N-undécylènoyl phénylalanine inhibe complètement l'activité de la protéine kinase A contrairement à l'acide kojique ou à l'arbutine.

### 4 - Etude de l'activité de la tyrosinase phosphorylée

On a comparé l'influence du N-undécylènoyl phénylalanine, de l'hydroquinone, de l'acide kojique et de l'arbutine sur l'activité de la tyrosinase phosphorylée en mesurant la transformation de L-Tyrosine en L-DOPA et DOPAquinone qui est un produit coloré quantifiable par spectrophotométrie (à 490 nm).

La tyrosinase utilisée est un produit commercial extrait de champignons.

Le N-undécylènoyl phénylalanine, l'hydroquinone, l'arbutine et l'acide kojique sont dilués à une concentration de 10 mg / ml dans une solution aqueuse de soude décinormale. Il sont testés chacun séparément aux concentrations de 0,1 mg / ml et 1 mg / ml. La soude n'a pas d'effet sur le paramètre étudié.

La tyrosinase à 66,66 UI / ml est incubée, en présence ou en absence de ces produits, et en présence de tyrosine à 0,2 mM, pendant 10 minutes à 37°C.

Des cultures témoins sont incubées, en absence de produit, et en présence de tyrosinase et de L-tyrosine. Chaque essai est réalisé trois fois.

Après 10 minutes d'incubation, la quantité d'histone de DOPAquinone formée est mesurée avec un spectrophotomètre à 490nm.

Les résultats consignés dans le tableau suivant, sont les moyennes des trois essais réalisées pour chacun des produits; Ils sont exprimés en U/I d'activité tyrosinase et en pourcentage d'inhibition de l'activité enzymatique par rapport au témoin.

| Produits testés | Pourcentage d'inhibition de l'activité de la tyrosinase par les produits testés par rapport au témoin | |
|---|---|---|
| | à 0,1 mg / ml | à 1 mg / ml |
| Hydroquinone | 78 | 80 |
| Arbutine | 73 | 80 |
| acide kojique | 76 | 80 |
| N-undécylènoyl phénylalanine | 80 | 100 |

Les résultats mettent en évidence qu'aux concentrations de 0,1 mg / ml et de 1 mg / ml, l'ensemble des produits testés inhibe de façon significative, l'activité de la tyrosinase. Cependant l'activité inhibitrice de l'undécylènoyl phénylalanine est supérieure à celle des autres produits testés.

### 5 - Etude de l'activité dépigmentante dans des cultures de mélanocytes B16/F1

On a comparé l'influence du N-undécylènoyl phénylalanine, de l'hydroquinone, de l'acide kojique et de l'arbutine sur la production de mélanine intracellulaire et de mélanine extracellulaire, dans des cultures de mélanocytes B16/F1 et sur l'activité de la tyrosinase phosphorylée.

Les mélanocytes murins de la lignée B16/F1 sont ensemencés dans des plaques de culture de 96 puits à la densité de 1500 cellules/puits. Les cellules sont cultivées dans un milieu de culture (milieu MCM) à 37°C dans une atmosphère humide contenant 5 % de CO₂. Les cellules sont utilisées à 60% de confluence, soit 4 jours après l'ensemencement.

Le milieu MCM a la composition suivante : Milieu DMEM (Dulbecco's Modified Eagle's Medium) contenant 4,5 g/l de glucose additionné de L-Glutamine (2 mM), de pénicilline (50 UI/ ml), de streptomycine (50 µg / ml) et de sérum de veau foetal (10 % v/v).

Le N-undécylènoyl phénylalanine est dilué à 4 mg/ml dans une solution aqueuse de soude décinormale. Il est testé à 40 µg / ml dans le milieu MCM. La soude n'a pas d'effet sur les paramètres analysés.

L'hydroquinone est testée à 5 µg / ml dans le milieu MCM. Compte tenu de sa toxicité, elle n'est pas testée à 40 µg / ml.

L'arbutine et l'acide kojique sont testés à 40 µg / ml dans le milieu MCM.

Les cultures de mélanocytes sont incubées en présence du produit à l'essai ou des produits de référence pendant 72 heures à 37°C, dans une atmosphère humide contenant 5 % de CO₂.

Des cultures témoins sont incubées, en absence de produit, dans le milieu MCM. Ces cultures témoins sont réalisées sur chaque plaque de culture.

Chaque essai est réalisé six fois.

### 5.1 - Mesure du contenu extracellulaire en mélanine

Après 72 heures d'incubation, les milieux d'incubation des cellules (n=6) sont prélevés et stockés à -80°C jusqu'au moment de l'évaluation des effets. La mélanine extracellulaire est quantifiée par spectrophotométrie à 450 nm. Une gamme d'étalonnage de mélanine est réalisée en parallèle.

Les résultats sont exprimés en µg / ml de mélanine extracellulaire et en pourcentage d'inhibition par rapport au groupe témoin.

| Produits testés | Mélanine extracellulaire (témoin : 43 ± 11 µg / ml) | Inhibition de la production de mélanine extracellulaire. |
|---|---|---|
| Produits testés | Mélanine extracellulaire (témoin : 43 ± 11 µg / ml) | Inhibition de la production de mélanine extracellulaire. |
| Hydroquinone à 5 µg / ml | 7 ± 1 µg / ml | 85% |
| Arbutine à 40 µg / ml | 23 ± 6 µg / ml | 47 % |
| acide kojique à 40 µg / ml | 13 ± 2 µg / ml | 70 % |
| N-undécylènoyl phénylalanine à 40 µg / ml | 12 ± 1 µg / ml | 72 % |

### 5.2 - Mesure du contenu intracellulaire en mélanine

Après 72 heures d'incubation, une partie des tapis cellulaires (n = 3) est rincée avec du tampon phosphate salin (PBS ; pH = 7,4) dont la composition est la suivante : NaCl : 8 g/l ; Na₂HPO₄ : 1,15 g/l ; KH₂PO₄ : 0,2g/l ; KCl : 0,2 g/l ; CaCl₂ : 0,1g/l ; MgCl₂ : 0,1 g/l. La mélanine intracellulaire est solubilisée par une incubation, sous agitation, pendant 30 minutes à température ambiante en présence de soude décanormale.

La mélanine intracellulaire est quantifiée par spectrophotométrie à 450 nm. Une gamme d'étalonnage de mélanine est réalisée en parallèle.

Les résultats sont exprimés en µg / ml de mélanine intracellulaire et en pourcentage d'inhibition par rapport au groupe témoin.

| Produits testés | Mélanine intracellulaire obtenue (témoin : 20 ± 4 µg / ml) | Inhibition de la production de mélanine intracellulaire. |
|---|---|---|
| Hydroquinone à 5 µg / ml | 0,2 ± 0,1 µg / ml | 100 % |
| Arbutine à 40 µg / ml | 16 ± 2 µg / ml | 19 % |
| acide kojique à 40 µg 1 ml | 17 ± 1 µg / ml | 17 % |
| Produits testés | Mélanine intracellulaire obtenue (témoin : 20 ± 4 µg / ml) | Inhibition de la production de mélanine intracellulaire. |
| N-undécylènoyl phénylalanine à 40 µg / ml | 7 ± 3 (g / ml | 66 % |

### 5.3 - Mesure de l'activité de la tyrosinase phosphorylée

Après 72 heures d'incubation, la seconde partie des tapis cellulaires (n=3) est rincée avec du PBS. Les cellules sont lysées par du Triton™ X100 à une concentration de 0,1 % (p / v) pendant 30 minutes à température ambiante. L'activité de la tyrosinase endogène est évaluée par addition de 0,1% (p / v) de L-DOPA, puis incubation de 3 heures à 37°C à l'abri de l'air et de la lumière. La DOPAquinone, formée par la réaction entre la tyrosinase et la L-DOPA, est mesurée par spectrophotométrie à 450 nm. Une gamme d'étalonnage de tyrosinase purifiée est réalisée en parallèle.

Les résultats sont exprimés en UI / ml d'activité tyrosinase et en pourcentage d'inhibition par rapport au groupe témoin:

| Produits testés | Activité tyrosinase (témoin : 9,8 ± 0,3 UI / ml) | Inhibition de l'activité de la tyrosinase. |
|---|---|---|
| Hydroquinone à 5 µg / ml | 3,0 ± 0,3 UI / ml | 69 % |
| Arbutine à 40 µg / ml | 7.6 ± 1,1 UI / ml | 23 % |
| acide kojique à 40 µg / ml | 6,8 ± 0,1 UI / ml | 31 % |
| N-undécylènoyl phénylalanine à 40 µg / ml | 3.2 ± 0.6 UI / ml | 67% |

### 5.4 - Mesure du contenu intracellulaire en protéines

Ce dosage permet d'évaluer la cytotoxicité des produits testés. Il est réalisé dans les lysats cellulaires préparés comme décrit au paragraphe précédent.

Le dosage des protéines est réalisé selon la méthode au bleu de Coomassie décrite par : "Bradford M. ; Anal. Biochem., 72, 1976, 248-254". La mesure se fait par spectrophotométrie à 640 nm. Une gamme d'étalonnage d'albumine sérique bovine (BSA) est réalisée en parallèle.

Les résultats sont exprimés en mg / ml de protéines et en pourcentage d'inhibition par rapport au groupe témoin.

| Produits testés | protéines totales (témoin : 0,45 ± 0,01) | Inhibition de la quantité de protéines. |
|---|---|---|
| Hydroquinone à 5 µg / ml | 0,28 ± 0,01 mg / ml | 38 % |
| Arbutine à 40 µg / ml | 0,43 ± 0,01 mg / ml | 5 % |
| acide kojique à 40 µg 1 ml | 0,41 ± 0,02 mg / ml | 10 % |
| N-undécylènoyl phénylalanine à 40 µg / ml | 0,38 ± 0,01 mg / ml | 17 % |

### 5.5 - Analyse des résultats

Après 72 heures d'incubation, l'hydroquinone, testée à 5 µg / ml, inhibe respectivement de 85% le contenu extracellulaire en mélanine, de 100% le contenu intracellulaire en mélanine et de 69% l'activité tyrosinase. Cependant l'effet dépigmentant de l'hydroquinone provient en partie de son effet cytotoxique car une diminution de 38% de la quantité totale de protéines est observée.

L'arbutine, testée à 40 µg / ml, inhibe respectivement de 47% le contenu extracellulaire en mélanine, de 19% le contenu intracellulaire en mélanine et de 23% l'activité tyrosinase. A cette concentration, l'arbutine n'a pas d'effet sur le contenu total en protéines.

L'acide kojique, testé à 40 µg / ml, inhibe respectivement de 70% le contenu extracellulaire en mélanine, de 17% le contenu intracellulaire en mélanine et de 31% l'activité tyrosinase. A cette concentration, l'acide kojique n'a pas d'effet significatif sur le contenu total en protéines.

Le N-undécylènoyl phénylalanine, testé à 40 µg / ml, inhibe respectivement de 72% le contenu extracellulaire en mélanine, de 66% le contenu intracellulaire en mélanine et de 67% l'activité tyrosinase. A cette concentration, le N-undécylènoyl phénylalanine diminue de 17% le contenu total en protéines.

Le N-undécylènoyl phénylalanine présente donc une activité dépigmentante mise en évidence par une diminution concomitante des contenus intra- et extracellulaire en mélanine et de l'activité tyrosinase. Son activité dépigmentante n'est pas liée, contrairement à celle de l'hydroquinone, à un effet cytotoxique. Il présente une activité dépigmentante supérieure à celles de l'arbutine et de l'acide kojique.

### 6 Etude de l'activité dépigmentante dans des épidermes humains reconstruits

On a comparé l'influence du N-undécylènoyl phénylalanine, de l'hydroquinone, de l'acide kojique et de l'arbutine sur la production de mélanine intracellulaire et de mélanine extracellulaire, dans des cultures de mélanocytes B16/F1 et sur la coloration des épidermes.

Des épidermes humains pigmentés (phototype IV) fournis par SKINETHIC de 0,63 cm², sont reconstruits à partir d'une co-culture de kératinocytes humains normaux (peau de l'avant bras, donneur de 3 ans, 2^{d} passage) et de mélanocytes humains normaux (peau de l'avant bras, donneur de 4 ans de phototype IV, 3^{ième} passage). Le rapport kératinocytes /mélanocytes est de 10:1. Les cocultures sont ensemencées sur des filtres inertes de polycarbonate. Elles sont cultivées pendant 10 jours dans le milieu fourni par SKINETHIC constitué du milieu MCDB 153, additionné de 5 µg / ml d'insuline, de 1,5 mM de calcium et de facteurs de croissance.

Pour ces essais, les produits sont testés après avoir été incorporés dans une formulation cosmétique consistant en émulsion comprenant une phase aqueuse, 10 % en poids d'une phase grasse (Lanol™ 1688), 2% en poids d'un émulsionnant (SIMUGEL™ EG), 0,5% en poids de conservateurs (0,3% de SEPICIDE™ HB + 0,2% de SEPICIDE™ Cl). après incorporation du principe actif, la formulation est ajusté à pH = 5,5.

Le N-undécylènoyl phénylalanine, l'arbutine et l'acide kojique y sont incorporés à chaud (75°C) dans une proportion de 1 % ou 3 % en poids par volume (p / v).

A cause de sa toxicité, l'hydroquinone y est incorporée à une concentration de 0,1 % en poids par volume (p / v).

Les épidermes sont mis en culture dans des plaques de 6 puits contenant 1 ml du milieu décrit ci-dessus. Ils sont incubés à 37°C dans une atmosphère humide contenant 5% de CO₂.

Les formulations contenant les différents principes actifs, sont appliqués à la surface des épidermes, à raison de 2 µl / épiderme, à l'aide d'un inoculateur bactériologique stérile. L'application est réalisée tous les jours pendant 4 jours consécutifs. Le milieu d'incubation des épidermes reconstruits est renouvelé tous les jours, pendant 4 jours consécutifs.

Des épidermes témoins, sont traités, avec une formulation sans principe actif. Chaque essai est réalisé deux fois.

### 6.1 - Mesure chromamétrique de la pigmentation des épidermes.

Trois jours après la dernière application topique, la couleur des épidermes est évaluée par un chromamètre (MINOLTA) en mesurant les paramètres L*, a* et b* suivants :
L* est l'indice de luminosité. Un produit dépigmentant doit augmenter ce paramètre ;
a* représente le spectre de couleur du bleu au vert. Un produit dépigmentant doit diminuer ce paramètre ;
b* : représente le spectre de couleur du rouge au jaune. Un produit dépigmentant doit diminuer ce paramètre.

Les résultats sont exprimés en unité arbitraire (UA) de chaque paramètre et en pourcentage du groupe témoin.

| Produits testés | L* | | a* | | b* | |
|---|---|---|---|---|---|---|
| | témoin : 41,38 ± 0,69 UA | | témoin : 10.23 ± 0,51 UA | | témoin : 7,54 ± 0,00 UA | |
| | UA | % | UA | % | UA | % |
| Hydroquinone formulé à 0,1 % (p / v) | 40,59 ± 2,41 | -9 | 10,19 ± 1,21 | 0 | 7,73 ± 0,03 | +3 |
| Arbutine formulé à 1 % (p / v) | 40,48 ± 0,85 | +2 | 10,02 ± 0,13 | -2 | 7,64 ± 0,19 | +1 |
| Arbutine formulé à 3 % (p / v) | 41,13 ± 0,54 | +1 | 9,86 ± 0,18 | -4 | 7,73 ± 0,34 | +3 |
| acide kojique formulé à 1 % (p / v) | 40,11 ± 1,41 | +3 | 10,90 ± 0,51 | +7 | 7,67 ± 0,03 | +3 |
| acide kojique formulé à 3% (p/v) | 41,69 ± 0,65 | 0 | 9,36 ± 1,02 | -8 | 7,13 ± 0,09 | -5 |
| N-undécylènoyl phénylalanine formulé à 1 % (p/v) | 41,34 ± 1,15 | 0 | 9,01 ± 1,33 | -12 | 5,96 ± 0,42 | -21 |
| N-undécylènoyl phénylalanine formulé à 3% (p/v) | 45,228 ± 1,49 | +9 | 8,78 ± 0,52 | -14 | 5,38 ± 0,06 | -29 |

### 6.2 - Mesure du contenu intracellulaire en mélanine

Trois jours après la dernière application topique et après la mesure chromamétrique, la mélanine intracellulaire est extraite des épidermes par une incubation de 45 minutes à 100°C dans du Soluène™ 350 (200 µl/épiderme), comme décrit dans : "Ozeki H., Ito, S., Wakamatsu K., Hirobe T. ; J. Invest. Dermatol. 105, 1995, 361-366. Les échantillons sont centrifugés pendant 10 minutes à 10 000 rpm.

La mélanine intracellulaire extraite est mesurée par spectrophotométrie à 500 nm. Une gamme d'étalonnage de mélanine est réalisée en parallèle.

Les résultats sont exprimés en mg / ml de mélanine intracellulaire et en pourcentage d'inhibition par rapport au groupe témoin.

| Produits testés | Mélanine intracellulaire (témoin : 347 ± 2 µg / ml) | Inhibition de la quantité de mélanine intracellulaire. |
|---|---|---|
| Hydroquinone formulé à 0,1% (p/v) | 317 ± 0 µg / ml | -9 % |
| Arbutine formulé à 1% (p/v) | 242 ± 46 µg / m | -28% |
| Arbutine à 3% (p/v) | 263 ± 16 µg / ml | -24% |
| acide kojique à 1 %(p/v) | 273 ± 2 µg/ml | -21 % |
| acide kojique à 3%(p/v) | 234 ± 19 µg/ml | -33% |
| N-undécylènoyl phénylalanine à 1 %(p/v) | 264 ± 9 µg/ml | -24 % |
| N-undécylènoyl phénylalanine à 3%(p/v) | 264 ± 11 µg/ ml | -24 % |

### 6.3 - Analyse des résultats

L'hydroquinone, testée en application topique à une concentration de 0,1 % (p / v) dans une émulsion, n'a d'effet significatif ni sur les paramètres chromamétriques L*, a* et b* ni sur le contenu en mélanine des épidermes humains reconstruits. L'absence d'effet dépigmentant de l'hydroquinone est due soit à la faible concentration testée délibérément choisie comme non cytotoxique, soit à la durée courte du traitement.

L'arbutine, testée en application topique à 1 et 3% (p / v) dans une émulsion, n'a pas d'effet significatif sur les paramètres chromamétriques L*, a* et b*. Par contre il inhibe respectivement de 28 et 24% le contenu en mélanine des épidermes humains reconstruits.

L'acide kojique, testé en application topique à 1 et 3 % (p / v) dans une émulsion, n'a pas d'effet significatif sur les paramètres chromamétriques L*, a* et b* Par contre, elle inhibe respectivement de 21 et 33% le contenu en mélanine des épidermes humains reconstruits.

Le N-undécylènoyl phénylalanine, testé à en application topique à 1 % (p / v) dans une émulsion, inhibe de 15% le paramètre de couleur b* et de 24% le contenu en mélanine des épidermes humains reconstruits.

A une concentration de 3 % (p / v), le N-undécylènoyl phénylalanine augmente de 9% le paramètre L*, et diminue de façon concomitante de 14% le paramètre de couleur a*, de 29% le paramètre de couleur b* et de 24% le contenu en mélanine des épidermes reconstruits.

Ces essais sur épidermes reconstruits, font apparaître que le N-undécylènoyl phénylalanine présente une activité dépigmentante améliorée par rapport à celle des produits de référence, en ce qu'elle influe tant sur les paramètres chromamétriques que sur le taux de mélanine intracellulaire.

### 7 - Conclusion

L'ensemble des résultats obtenus dans cette étude met en évidence une forte activité dépigmentante du N-undécylènoyl phénylalanine. Cette dernière est quantifiée aussi bien dans des cultures de mélanocytes que dans un modèle 3D composé d'épidermes humains reconstruits. Contrairement aux produits de référence, l'activité dépigmentante du N-undécylènoyl phénylalanine met en jeu les récepteurs MC1 R. Le N-undécylènoyl phénylalanine est un antagoniste du récepteur à MC1 R et il inhibe toutes le étapes du cycle de l'α-MSH impliquée dans la mélanogénèse.

### Exemples de formulation cosmétiques

### Exemple 2 : Emulsion-Soin éclaircissant pour peau mature

| | |
|---|---|
| MONTANOV™ 202 | 02, 00 % |
| MONTANOV™ 68 | 02,00 % |
| Caprylic capric triglycérides | 10,00 % |
| Squalane | 10,00 % |
| Eau | QSP 100 % |
| N-undécylènoyl phénylalanine | 01,00 % |
| SEPIGEL™ 305 | 00,70 % |
| Magnésium ascorbyl phosphate | 02,00 % |
| SEPICIDE™ HB | 00,30 % |
| SEPICIDE™ CI | 00,20 % |
| Parfum | 00,50 % |

### Exemple 3 : Emulsion-Soin raffermissant éclaircissant

| | |
|---|---|
| MONTANOV™ 202 | 03, 00 % |
| Soude à 24 % | 00,06 % |
| Ethyl hexyl méthoxycinnamate | 06,00 % |
| LANOL™1688 | 08,00 % |
| Benzophénone-3 | 04,00 % |
| Eau | QSP 100 % |
| N-undécylènoyl phénylalanine | 02,00 % |
| SIMULGEL™ NS | 00,50 % |
| Sepilift™ DPHP | 00,50 % |
| Diméthicone | 02,00 % |
| Cyclométhicone | 02,00 % |
| Arbutine | 0.3 % |
| SEPICIDE™ HB | 00,30 % |
| SEPICIDE™ CI | 00,20 % |
| Parfum | 00,10 % |

### Exemple 4 : Gel-crème éclaircissant aux alphahydroxyacides

| | |
|---|---|
| Hydroxyéthylcellulose | 00,80 % |
| Ethylhexyl octanoate | 05,00 % |
| Sodium lactate à 60 % | 14,00 % |
| Eau | QSP 100 % |
| N-undécylènoyl phénylalanines | 03,00 % |
| SEPIGEL™ 305 | 04,20 % |
| SEPICIDE™ HB | 02,00 % |
| SEPICIDE™ Cl | 03,00 % |
| Parfum | 00,10 % |

### Exemple 5 : Emulsion- Soin éclaircissant

| | |
|---|---|
| MONTANOV™ L | 01, 00 % |
| Cetyl alcool | 02,00 % |
| Isodecyl neopentanoate | 12,00 % |
| Cetearyl octanoate | 10,00 % |
| Glycérine | 03,00 % |
| Eau | QSP 100 % |
| N-undécylènoyl phénylalanine | 01,00 % |
| SIMULGEL™ EG | 02,00 % |
| Acide kojique | 01,00 % |
| SEPICIDE™ HB | 00,30 % |
| SEPICIDE™ CI | 00,20 % |
| Parfum | 00,10 % |

### Exemple 6 : Lotion éclaircissante

| | |
|---|---|
| ORAMIX™ CG110 | 05,00 % |
| KATHON™ CG | 00,08 % |
| Eau | QSP 100 % |
| N-undécylènoyl phénylalanine | 01,00 % |
| Parfum | 00,10 % |

Cette lotion peut être vendue en flacons ou imprégnée sur des lingettes.

Les définitions des produits commerciaux utilisés dans les exemples sont les suivantes :
SEPILIFT™ DHP (Dénomination INCI: Dipalmitoyl hydroxyproline), commercialisé par la société SEPPIC.
SEPICIDE™ HB est un mélange conservateur comprenant du Phenoxyethanol, du méthylparaben, de l'éthylparaben, du propylparaben et du butyl paraben, commercialisé par la société SEPPIC.
SEPICIDE™ Cl est de l'imidazolidinyl urée, commercialisé par la société SEPPIC.
SEPICALM™ VG (Dénomination INCI : sodium palmitoyl proline et extrait de fleur de nénuphar), commercialisé par la société SEPPIC.
KATHON™ CG (Dénommination INCI: méthyl isothiazolinone / Méthyl chloroisothiazolinone).
SIMULGEL™ EG est un latex inverse de copolymère (dénomination INCI : Sodium acrylate/Sodium acryloyldimethyl taurate copolymer et Isohexadecane et Polysorbate 80) commercialisé par la société SEPPIC.
SIMULGEL™ NS est un latex inverse de copolymère (dénomination INCI: hydroxyethylacrylate/Sodium acryloyldimethyl taurate copolymer et squalane et Polysorbate 60) commercialisé par la société SEPPIC.
LANOL™ 1688 est du cétéaryl ethylhexanoatel commercialisé par la société SEPPIC.
SEPIGEL™ 305 est un latex inverse de polymère (dénomination INCI :: polyacrylamide et isoparaffine en C13-C14 et laureth 7).
MONTANOV™ L est un agent émulsionnant à base d'alcool en C14-C22 et d'alkyl polyglucoside en C12-C20.
MONTANOV™ 68 est un agent émulsionnant à base d'alcool cétéarylique et cétéaryl polyglucoside
MONTANOV™ 202 est un agent émulsionnant à base d'alcool arachidylique d'alcool béhènylique et d'arachidyl polyglucoside.

## Revendications

1. Utilisation pour éclaircir la peau non-thérapeutique de N-(ω-undécylènoyl) phénylalanine de formule (la) inactivant la protéine Kinase A dans une composition cosmétique contenant un milieu cosmétiquement acceptable.

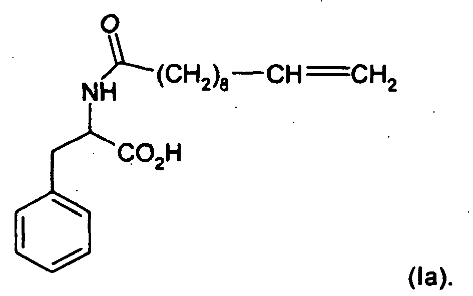

2. Utilisation telle que définie à la revendication 1 de N-(ω-undécylènoyl) phénylalanine de formule (Ia), **caractérisée en ce que** le composé de formule (la) inactivant la protéine Kinase A, inactive aussi l'adénylate cyclase.

3. Utilisation telle que définie à la revendication 1 de N-(ω-undécylènoyl) phénylalanine de formule (Ia) **caractérisée en ce que** le composé de formule (Ia) inactivant la protéine Kinase A et l'adénylate cyclase, est un composé présentant une affinité vis à vis du récepteur de la Mélanocyte Spécifique Hormone (α-MSH).

4. Procédé de traitement non thérapeutique de la peau destiné à l'éclaircir, **caractérisé en ce que** l'on administre au sujet, une composition cosmétique contenant un milieu cosmétiquement acceptable et une quantité efficace de N-(ω-undécylènoyl) phénylalanine de formule (la) inactivant la protéine Kinase A.

5. Procédé tel que défini à la revendication 4, dans lequel ladite composition cosmétique contient entre 0,01 % et 10 % de son poids total, plus particulièrement entre 0,1 % à 5 % de son poids total et tout particulièrement, entre 1 % et 5 % de son poids total de N-(ω-undécylènoyl) phénylalanine de formule (la) inactivant la protéine Kinase A.

6. Procédé tel que défini à l'une des revendications 4 ou 5, **caractérisé en ce que** le N-(ω-undécylènoyl) phénylalanine de formule (la) inactivant la protéine Kinase A, inactive aussi l'adénylate cyclase.

7. Procédé tel que défini à la revendication 6, **caractérisé en ce que** le composé de formule (Ia) inactivant la protéine Kinase A et l'adénylate cyclase, présente une affinité vis à vis du récepteur de la Mélanocyte Spécifique Hormone.

8. Utilisation de N-(ω-undécylènoyl) phénylalanine de formule (la) inactivant la protéine Kinase pour la préparation d'une composition pharmaceutique destinée à éclaircir la peau.

9. Utilisation telle que définie à la revendication 8 de N-(ω-undécylènoyl) phénylalanine de formule (Ia) **caractérisée en ce que** la composition pharmaceutique préparée contient entre 0,01 % et 10 % de son poids total, plus particulièrement entre 0,1 % à 5 % de son poids total et tout particulièrement, entre 1 % et 5 % de son poids total de N-(ω-undécylènoyl) phénylalanine de formule (la) inactivant la protéine Kinase A.

10. Utilisation telle que définie à l'une des revendications 8 ou 9 de N-(ω-undécylènoyl) phénylalanine de formule (Ia) **caractérisée en ce que** le composé de formule (Ia) inactivant la protéine Kinase A, inactive aussi l'adénylate cyclase.

11. Utilisation telle que définie à la revendication 10 de N-(ω-undécylénoyl) phénylalanine de formule (Ia) **caractérisée en ce que** le composé de formule (la) inactivant la protéine Kinase A et l'adénylate cyclase, présente une affinité vis à vis du récepteur de la Mélanocyte Spécifique Hormone.

12. Emulsion cosmétique **caractérisée en ce qu'**elle contient entre 0,01 % et 10 % de son poids total, plus particulièrement entre 0,1 % à 5 % de son poids total, et tout particulièrement entre 1 % et 5 % de son poids total du composé de formule (Ia).

## Claims

1. Nontherapeutic use for lightening the skin of N-(ω-undecylenoyl)phenylalanine of formula (Ia): 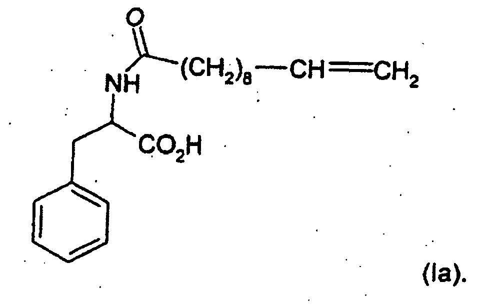 which inactivates protein kinase A, in a cosmetic composition comprising a cosmetically acceptable medium.

2. Use as defined in Claim 1 of N-(ω-undecylenoyl)-phenylalanine of formula (Ia), **characterized in that** the compound of formula (Ia), which inactivates protein kinase A, also inactivates adenylate cyclase.

3. Use as defined in Claim 1 of N-(ω-undecylenoyl)-phenylalanine of formula (Ia), **characterized in that** the compound of formula (Ia), which inactivates protein kinase A and adenylate cyclase, is a compound exhibiting an affinity with regard to the melanocyte-stimulating hormone (α-MSH) receptor.

4. Method for the nontherapeutic treatment of the skin intended to lighten it, **characterized in that** a cosmetic composition comprising a cosmetically acceptable medium and an effective amount of N-(ω-undecylenoyl)phenylalanine of formula (Ia), which inactivates protein kinase A, is administered to the subject.

5. Method as defined in Claim 4, in which the said cosmetic composition comprises between 0.01% and 10% of its total weight, more particularly between 0.1% and 5% of its total weight and very particularly between 1% and 5% of its total weight of N-(ω-undecylenoyl)phenylalanine of formula (Ia) which inactivates protein kinase A.

6. Method as defined in either of Claims 4 and 5, **characterized in that** the N-(ω-undecylenoyl)-phenylalanine of formula (Ia), which inactivates protein kinase A, also inactivates adenylate cyclase.

7. Method as defined in Claim 6, **characterized in that** the compound of formula (Ia), which inactivates protein kinase A and adenylate cyclase, exhibits an affinity with regard to the melanocyte-stimulating hormone receptor.

8. Use of N-(ω-undecylenoyl)phenylalanine of formula (Ia), which inactivates protein kinase A, in the preparation of a pharmaceutical composition intended to lighten the skin.

9. Use as defined in Claim 8 of N-(ω-undecylenoyl)-phenylalanine of formula (Ia), **characterized in that** the pharmaceutical composition prepared comprises between 0.01% and 10% of its total weight, more particularly between 0.1% and 5% of its total weight and very particularly between 1% and 5% of its total weight of N-(ω-undecylenoyl)-phenylalanine of formula (Ia) which inactivates protein kinase A.

10. Use as defined in either of Claims 8 and 9 of N-(ω-undecylenoyl)phenylalanine of formula (Ia), **characterized in that** the compound of formula (Ia), which inactivates protein kinase A, also inactivates adenylate cyclase.

11. Use as defined in Claim 10 of N-(ω-undecylenoyl)-phenylalanine of formula (Ia), **characterized in that** the compound of formula (Ia), which inactivates protein kinase A and adenylate cyclase, exhibits an affinity with regard to the melanocyte-stimulating hormone receptor.

12. Cosmetic emulsion, **characterized in that** it comprises between 0.01% and 10% of its total weight, more particularly between 0.1% and 5% of its total weight and very particularly between 1% and 5% of its total weight of the compound of formula (Ia).

## Patentansprüche

1. Nicht-therapeutische Verwendung zur Klärung der Haut von N-(ω-Undecylenoyl)-phenylalanin der Formel (Ia), das die Proteinkinase A inaktiviert, in einer Kosmetikzusammensetzung, die ein kosmetisch annehmbares Medium enthält

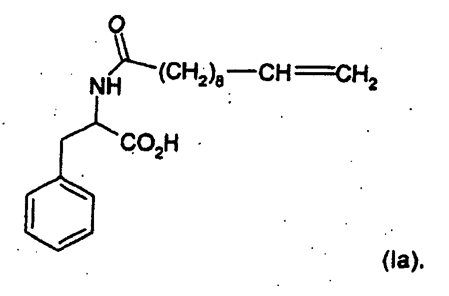

2. Verwendung nach Anspruch 1 von N-(ω-Undecylenoyl)-phenylalanin der Formel (Ia), **dadurch gekennzeichnet, dass** die Verbindung der Formel (Ia), welche die Proteinkinase A inaktiviert, außerdem die Adenylatcyclase inaktiviert.

3. Verwendung nach Anspruch 1 von N-(ω-Undecylenoyl)-phenylalanin der Formel (Ia), **dadurch gekennzeichnet, dass** die Verbindung der Formel (Ia), welche die Proteinkinase A und die Adenylatcyclase inaktiviert, eine Verbindung mit einer Affinität für den Rezeptor des melanozytenspezifischen Hormons (α-MSH) ist.

4. Verfahren zur nicht-therapeutischen Behandlung der Haut, um sie zu klären, **dadurch gekennzeichnet, dass** man dem Individuum eine Kosmetikzusammensetzung verabreicht, die ein kosmetisch annehmbares Medium und eine wirksame Menge an N-(ω-Undecylenoyl)-phenylalanin der Formel (Ia), das die Proteinkinase A inaktiviert, enthält.

5. Verfahren nach Anspruch 4, wobei die Kosmetikzusammensetzung zwischen 0,01% und 10%, bezogen auf ihr Gesamtgewicht, insbesondere zwischen 0,1% und 5%, bezogen auf ihr Gesamtgewicht, und ganz besonders zwischen 1% und 5%, bezogen auf ihr Gesamtgewicht, an N-(ω-Undecylenoyl)-phenylalanin der Formel (Ia), das die Proteinkinase A inaktiviert, enthält.

6. Verfahren nach einem der Ansprüche 4 oder 5, **dadurch gekennzeichnet, dass** das N-(ω-Undecylenoyl)-phenylalanin der Formel (Ia), das die Proteinkinase A inaktiviert, außerdem die Adenylatcyclase inaktiviert.

7. Verfahren nach Anspruch 6, **dadurch gekennzeichnet, dass** die Verbindung der Formel (Ia), welche die Proteinkinase A und die Adenylatcyclase inaktiviert, eine Affinität für den Rezeptor des melanozytenspezifischen Hormons aufweist.

8. Verwendung von N-(ω-Undecylenoyl)-phenylalanin der Formel (Ia), das die Proteinkinase A inaktiviert, zur Herstellung einer pharmazeutischen Zusammensetzung zur Klärung der Haut.

9. Verwendung nach Anspruch 8 von N-(ω-Undecylenoyl)-phenylalanin der Formel (Ia), **dadurch gekennzeichnet, dass** die hergestellte pharmazeutische Zusammensetzung zwischen 0,01% und 10%, bezogen auf ihr Gesamtgewicht, insbesondere zwischen 0,1% und 5%, bezogen auf ihr Gesamtgewicht, und ganz besonders zwischen 1% und 5%, bezogen auf ihr Gesamtgewicht, an N-(ω-Undecylenoyl)-phenylalanin der Formel (Ia), das die Proteinkinase A inaktiviert, enthält.

10. Verwendung nach einem der Ansprüche 8 oder 9 von N-(ω-Undecylenoyl)-phenylalanin der Formel (Ia), **dadurch gekennzeichnet, dass** die Verbindung der Formel (Ia), welche die Proteinkinase A inaktiviert, außerdem die Adenylatcyclase inaktiviert.

11. Verwendung nach Anspruch 10 von N-(ω-Undecylenoyl)-phenylalanin der Formel (Ia), **dadurch gekennzeichnet, dass** die Verbindung der Formel (Ia), welche die Proteinkinase A und die Adenylatcyclase inaktiviert, eine Affinität für den Rezeptor des melanozytenspezifischen Hormons aufweist.

12. Kosmetische Emulsion, **dadurch gekennzeichnet, dass** sie zwischen 0,01% und 10%, bezogen auf ihr Gesamtgewicht, insbesondere zwischen 0,1% und 5%, bezogen auf ihr Gesamtgewicht, und ganz besonders zwischen 1% und 5%, bezogen auf ihr Gesamtgewicht, der Verbindung der Formel (Ia) enthält.
